# EUROPEAN PATENT SPECIFICATION

(11) **EP 1 582 231 B1**
(45) Date of publication and mention of the grant of the patent: **25.07.2012**
(21) Application number: 05252067.3
(22) Date of filing: 01.04.2005
(51) Int. Cl.: A61M 16/06

(54) **Improvements relating to respiratory masks**
Verbesserungen bezüglich Beatmungsmasken
Améliorations concernant des masques respiratoires

(30) Priority: 02.04.2004 GB 0407514; 22.10.2004 GB 0423584
(43) Date of publication of application: 05.10.2005
(73) Proprietor: Intersurgical AG, Vaduz (LI)
(72) Inventor: Miller, Andrew Neil, Bracknell RG12 9NJ (GB)
(74) Representative: Adamson Jones

(56) References cited:
- FR-A- 2 076 334
- GB-A- 2 336 547
- US-A- 4 971 051
- US-A- 5 469 842

## Description

This invention relates to respiratory masks suitable for delivering inhalation gases to the airways of a patient, and in particular to those respiratory masks that are suitable for use in oxygen and/or aerosol therapy.

Respiratory masks are used to supply inhalation gases, and possibly also atomised liquids such as drugs in solution, to the airways of a patient. In general, a gas is supplied to a respiratory enclosure defined by the respiratory mask and the face of the patient, and the patient inhales the inhalation gas from this respiratory enclosure. Conventional masks typically also have an inlet for the inhalation gas, and an outlet through which exhaled gas escapes the respiratory mask.

A conventional respiratory mask is relatively flexible, and is typically formed as a unitary component of polyvinylchloride (PVC) material. This unitary component defines a cavity, and typically has an outwardly-turned peripheral rim that is urged against the patient's face, about their nose and mouth, when fitted to the patient.

In order to maintain an effective seal between the peripheral rim of the respiratory mask and the patient's face, conventional respiratory masks have an elasticated strap that is placed about the patient's head so as to urge the respiratory mask against the face of the patient. In addition, an adjustable nose clip is typically fitted about the portion of the mask that surrounds the patient's nose so as to further improve the seal between the mask and the patient's face.

Respiratory masks such as those described above may be used for oxygen therapy in which oxygen is administered to a patient at a concentration greater than that of ambient air, and/or aerosol therapy in which a fine mist of a drug in solution is inhaled by a patient.

Conventional respiratory masks are generally formed from a single material. This material needs to be rigid enough for the mask to retain its shape during handling and keep the cavity open during use, but it also needs to be flexible enough for the mask to conform to the patient's face during use so as to form an effective seal. The material selected can only be a compromise between these differing requirements. Relatively flexible PVC material is generally used to form conventional respiratory masks so that these masks conform substantially to the contours of a patient's face, thereby providing a reasonably effective seal. However, the relative flexibility of such masks makes them more difficult to handle, less durable, and more prone to deformation in packaging and transit, than a more rigid mask. In addition, the use of PVC for the production of medical devices and respiratory masks is at present a growing issue in the medical industry.

FR 2 076 334 discloses a respiratory mask having a rigid mask body and a flexible elastomeric seal member which fits into the body and provides a seal formation for contact with a patient's face.

There has now been devised an improved respiratory mask which overcomes or substantially mitigates the above-mentioned and/or other disadvantages associated with the prior art.

According to the invention, there is provided a respiratory mask for delivering an inhalation gas to the airways of a patient, the respiratory mask comprising a mask body defining a cavity and being adapted to fit about the mouth and nose of the patient such that the inhalation gas can be inhaled by the patient from the cavity, an inlet port that is engageable with a supply of inhalation gas and in communication with the cavity, and one or more sealing components extending from the peripheral edge of the mask body and defining a contact surface that contacts the nose of the patient during use, one or more of the sealing components comprising a sealing member that defines the contact surface and a connecting member that connects the sealing member to the peripheral edge of the mask body, characterised in that the one or more sealing members include reinforcing formations that reduce the deformability of those parts of the sealing member in which they are formed, wherein the connecting member is resilient in form and is more deformable, in use, than the sealing member.

By "more deformable" is meant that the change in effective length as a function of applied force is greater for the connecting member than for the sealing member.

The connecting member may be formed of a more deformable material than the sealing member that it connects to the peripheral edge of the mask body. However, more preferably, the connecting member is formed of the same material as the sealing member. In this case, the connecting member preferably has a configuration that provides the greater deformability. In particular, the connecting member may be convoluted in form, for example in a corrugated or concertina-type configuration, so that the connecting member is more deformable than the sealing member.

The invention may enable different shapes and sizes of nose to be accommodated by the one or more sealing members without excessive deformation of the sealing members that could cause a break in the seal with the nose of the patient.

One or more sealing members that contact the chin of a patient are preferably connected to the peripheral edge of the mask body by a connecting member that is resilient in form and is more deformable than the one or more sealing members. As well as enabling different shapes and sizes of chin to be accommodated by the one or more sealing members, as discussed above in relation to sealing members that contact the nose, this feature enables the one or more sealing members that contact the chin of a patient to be readily moved relative to the mask body during use. In this way, the patient can talk whilst wearing the mask, and the sealing member will move with the patients mandible, thereby improving patient comfort.

In order to control the degree of deformation of each sealing member that occurs at different parts of the contact surface when the one or more sealing members are being deformed into conformity with the face of a patient, one or more of the sealing members include reinforcing formations, such as reinforcing ribs, that reduce the deformability of those parts of the sealing member in which they are formed. In a presently preferred embodiment, reinforcing formations are provided on the parts of the one or more sealing members that contact either side of a patient's nose so that the parts of the one or more sealing members that contact the bridge of a patient's nose deform to a greater degree.

Each sealing component is preferably formed from an elastomeric material, which is most preferably a Styrene-Ethylene-Butylene-Styrene (SEBS)-based thermoplastic elastomer. The respiratory mask is preferably manufactured using a so-called two-shot injection moulding process. In particular, the mask body is preferably injection moulded as a single component of a relatively rigid material, and the elastomeric material of the respiratory mask is then preferably injection moulded onto the surface of the mask body. The mask body and the elastomeric parts of the respiratory mask are bonded together by this process.

The mask body is preferably formed so that it maintains its shape when subjected to normal handling, packaging and storage conditions. The mask body is preferably formed from plastics material in an injection moulding process. Most preferably, the mask body is formed of polypropylene.

The mask body is preferably arranged so that the cavity accommodates the patient's nose and mouth when fitted to the patient. Most preferably, the mask body comprises a mouth portion and a nose portion, the depth of the cavity defined by the nose portion being greater then the depth of the cavity defined by the mouth portion. In addition, the nose portion is preferably tapered towards an apex that is shaped to fit around the bridge of the patient's nose.

The inlet port preferably comprises an opening in the wall of the cavity with a conventional tubular connector extending outwardly therefrom. Most preferably, the tubular connector extends from an opening in the nose portion of the mask body into a space adjacent to the mouth portion. In addition, the respiratory mask preferably includes openings in the wall of the cavity that allow exhaled gases to escape from the cavity of the respiratory mask, and also allow the intake of a sufficient quantity of air to satisfy the inspiratory requirements of the patient, as required during use. Where the respiratory mask is to be used for delivering a high concentration of an inhalation gas, such as oxygen, to a patient, the openings in the wall of the cavity will each generally include a valve, and a reservoir bag will generally be connected to the inlet port.

The respiratory mask according to the invention may include means for securing the mask to the patient, in use. Such means may include an elasticated cord or strap that is fitted around the patient's head to urge the respiratory mask against the face of the patient. The elasticated cord or strap may be formed of elastomeric material, and may therefore be formed integrally with the remainder of the respiratory mask using the two-shot injection moulding process. Alternatively, the elasticated cord or strap may be formed as a separate component.

Although the present invention may avoid the need for a nose clip to provide an effective seal about the patient's nose, the respiratory mask may include means for attaching a nose clip about the nose portion of the mask. Such means preferably includes a projection that is formed on the external surface of the mask, and which is engageable with the nose clip. Most preferably, the mask includes a projection on either side of the nose portion of the mask, each projection being engageable with one end of the nose clip.

Conventional nose clips comprise an opening at each end that each receive a projection, and include inwardly-projecting teeth that grip the projections within the openings. However, such nose clips have been found to suffer from the major disadvantage that they are liable to become disengaged from the mask.

The projections of the mask of the present invention are therefore preferably resilient in form, and are most preferably formed from elastomeric material. In addition, the nose clip preferably comprises a pair of arms at each end of the nose clip, each pair of arms slidably receiving and retaining a projection therebetween. In particular, each pair of arms preferably defines a receiving area therebetween, the receiving area having a holding portion within which the projection is located, in use, and an entrance portion of reduced dimension that acts to retain the projection within the holding portion. Most preferably, the entrance portion is tapered and includes a neck of reduced dimension immediately adjacent to the holding portion.

The invention will now be described in greater detail, by way of illustration only, with reference to the accompanying drawings, in which
Figure 1 is a perspective view of a first example of a respiratory mask;
Figure 2 is a front view of the first mask;
Figure 3 is a first perspective view of the rear of the first mask;
Figure 4 is a rear view of the first mask;
Figure 5 is a second perspective view of the rear of the first mask;
Figure 6 is a front view of a nose clip for use with the respiratory mask of Figures 1 to 5;
Figure 7 is a side view of the nose clip;
Figure 8 is a front view of an embodiment of a respiratory mask according to the invention;
Figure 9 is a side view of the embodiment of Figure 8;
Figure 10 is a cross-sectional view, along the line IX-IX of the embodiment in Figure 8; and
Figure 11 is an end view of the embodiment of Figure 8.

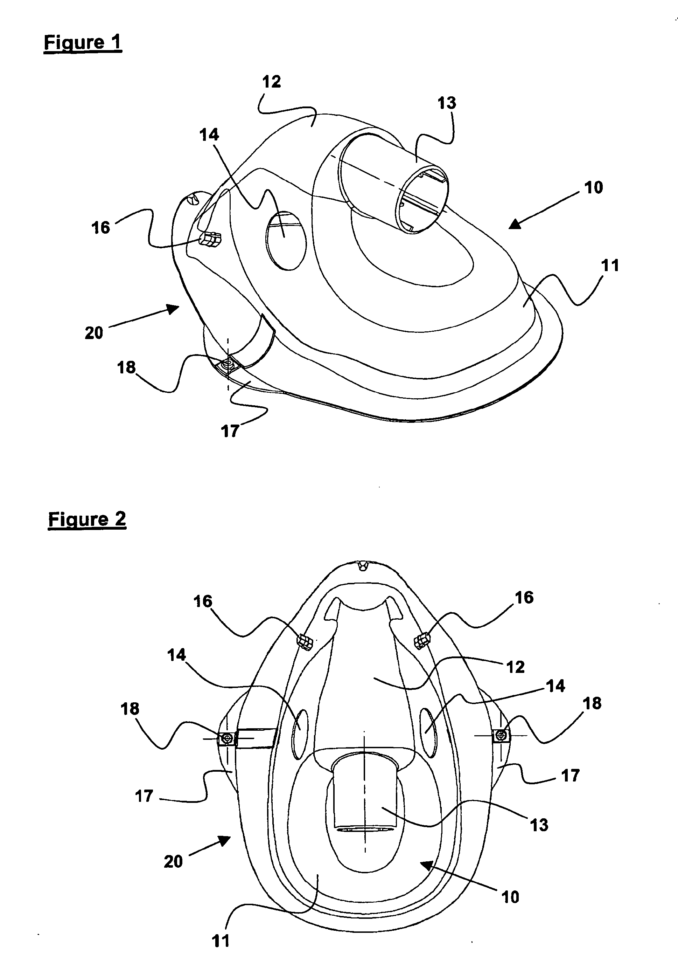

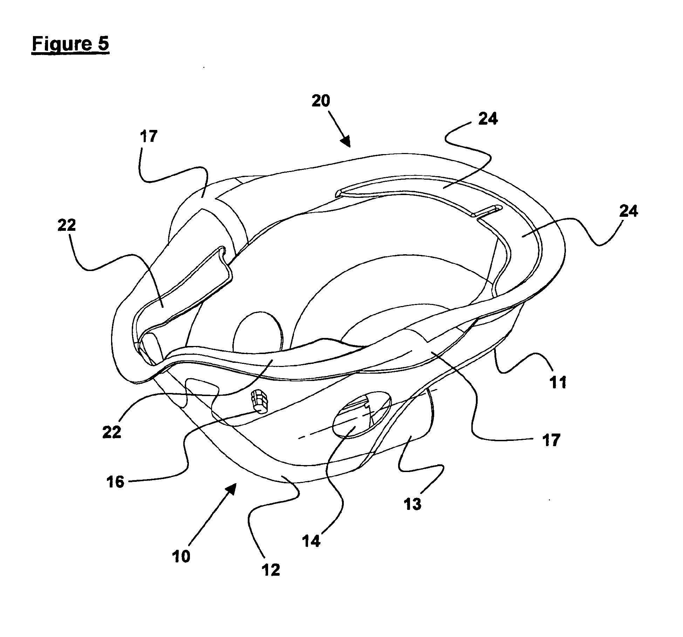

Figures 1 to 5 each show a first example of a respiratory mask. The respiratory mask comprises a mask body 10, formed from a suitably strong and relatively rigid plastics material, such as polypropylene, and a sealing flange 20 formed from a Styrene-Ethylene-Butylene-Styrene (SEBS)-based thermoplastic elastomer.

The respiratory mask is manufactured using a so-called two-shot injection moulding process. In particular, the mask body 10 is firstly injection moulded as a single component, and the sealing flange 20 is then injection moulded onto the surface of the mask body 10. The mask body 10 and the sealing flange 20 are bonded together by this process.

The mask body 10 defines a cavity from which an inhalation gas is delivered to a patient, and comprises a mouth portion 11 and a nose portion 12.

The sealing flange 20 is a unitary component that is bonded to, and extends from, the edge of the mask body 10. The sealing flange 20 is outwardly-turned, away from the opening defined by the edge of the mask body 10, such that the sealing flange 20 forms a substantially flat, and continuous, contact surface that is urged against the face of the patient during use. The sealing flange 20, and hence the contact surface, entirely surrounds the edge of the mask body 10. The elastomeric nature of the sealing flange 20 enables an effective seal to be formed between the contact surface of the respiratory mask and the face of the patient.

The mask body 10 is configured so that when the respiratory mask is fitted to a patient, the patient's mouth is accommodated within the mouth portion 11 of the mask body 10 and the patient's nose is accommodated within the nose portion 12 of the mask body 10. The nose portion 12 of the mask body 10 therefore has a greater depth than the mouth portion 11, and is tapered towards the upper end of the mask and hence the bridge of the patient's nose, as shown clearly in Figures 1 and 2.

The mask body 10 further comprises an inlet port 13 for connection to a supply of an inhalation gas, and a pair of exhalation openings 14. The inlet port 13 comprises an opening in a lower wall of the nose portion 12, and a tubular connector that extends downwardly (as viewed in Figure 2) from this opening into the space in front of the mouth portion 11. In use, a supply of an inhalation gas is connected to the tubular connector of the inlet port 13 so as to supply the inhalation gas to the cavity of the respiratory mask and hence the airways of the patient. The exhalation openings 14 are circular openings in the wall of the mask body 10 that allow exhaled gases to exit the cavity of the respiratory mask, and also allow the intake of air for inhalation, as required, when the inhalation volume exceeds the volume of the mask cavity and the inlet flow.

The sealing flange 20 is formed with a pair of outwardly extending wings 17, on either side of the respiratory mask, that each have an aperture 18 to which an elasticated strap (not shown in the Figures) is attached, in use. The elasticated strap extends between the pair of wings 17, and fits around the patient's head when the respiratory mask is fitted to the patient. In use, the elasticated strap is adjusted so that the respiratory mask is urged against the face of the patient with an appropriate force to ensure that an effective seal is formed between the contact surface of the respiratory mask and the patient's face, without causing excessive discomfort for the patient.

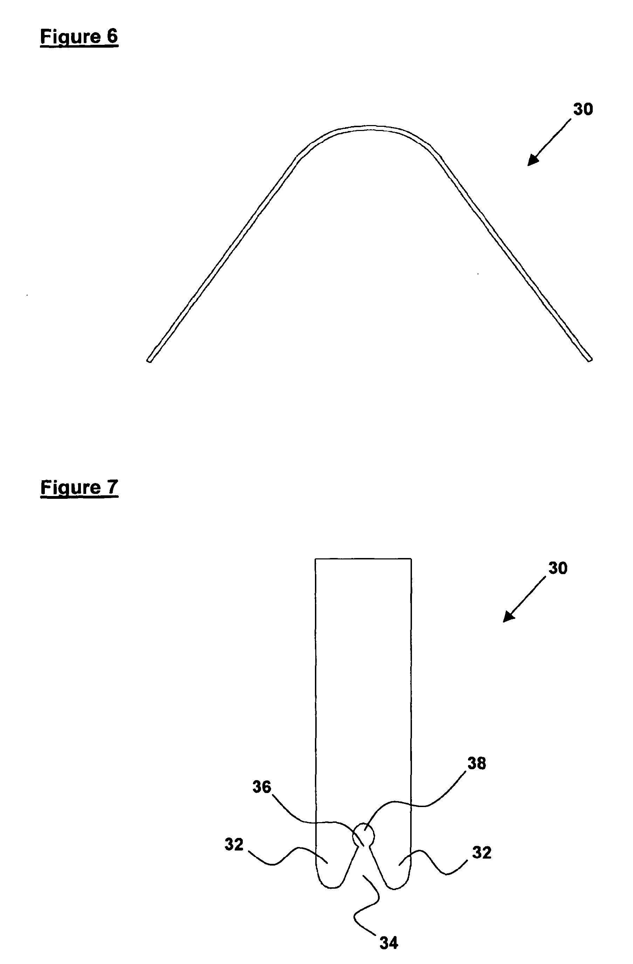

The respiratory mask of Figures 1 to 5 includes a pair of projections 16, situated on either side of the nose portion 12, that are adapted to be engaged by a nose clip 30 (shown in Figures 6 and 7). When engaged with the projections 16, in use, the nose clip 30 acts to clamp the nose portion 12 of the mask about the nose of the patient, thereby improving the seal between the respiratory mask and the face of the patient. The projections 16 are formed on the outwardly-facing surface of the sealing flange 20, and are formed from elastomeric material.

The nose clip 30 is shown in Figures 6 and 7, and comprises a band of aluminium that has been shaped so as to conform generally to the external surface of the nose portion 12 in a transverse direction. The nose clip 30 is deformable and able to retain its shape when deformed, thereby enabling the nose clip 30 to be deformed into a clamping arrangement with the nose portion 12 of the mask.

A pair of arms 32 extend from each end of the nose clip 30, and each pair of arms 32 is adapted to slidably receive and retain a projection 16 therebetween. Each pair of arms 32 defines a receiving area therebetween, the receiving area having a tapered entrance portion 34 with a narrow neck 36 at its inner end, and a circular holding portion 38 of increased dimensions relative to the neck 36. In use, the nose clip 30 is slidably engaged with each projection 16 by guiding each projection 16 through the tapered entrance portion 34 and neck 36, and into the circular holding portion 38, of the receiving area. The narrow neck 36 acts to prevent the projection 16 from slidably disengaging from the nose clip 30 during normal use.

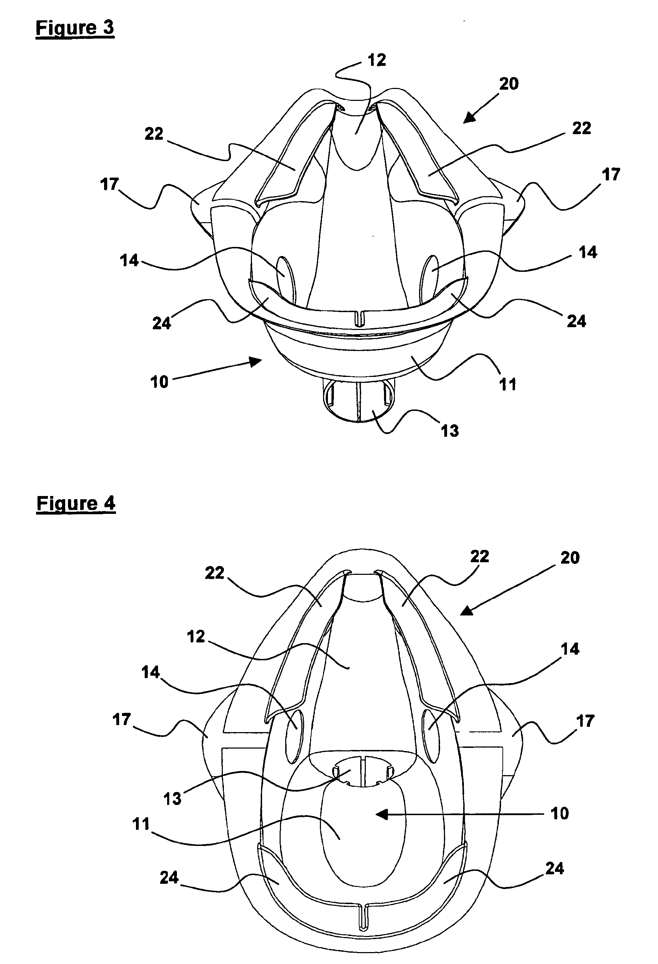

Even with the clamping action of a nose clip, it has been found for conventional respiratory masks that the seal between the contact surface and the face of the patient is least effective about the nose portion of the mask, which may cause gas to flow across the patient's eyes. The respiratory mask is therefore provided with a pair of upper sealing members 22 that are formed integrally with the sealing flange 20, and extend inwardly from the periphery of the mask, either side of the nose portion 12. Each upper sealing member 22 is resilient in form so that when the contact surface of the respiratory mask is urged against the patient's face, the upper sealing members 22 are deformed, and therefore urged against, the surface of the patient's nose. In this way, the upper sealing members 22 provide an additional seal between the respiratory mask and the face of the patient. At the upper end of the respiratory mask (as viewed in Figures 3 and 4), the upper sealing members 22 are spaced apart so as to accommodate the bridge of the patient's nose.

The upper sealing members 22 therefore improve the seal formed between the respiratory mask and the face of the patient. This may allow the respiratory mask to be used without a nose clip, allowing the mask to be fitted more easily and quicker to the patient.

Another problem addressed by the mask is that conventional face masks only fit faces having a size that is within a relatively narrow range. The respiratory mask according to the invention is therefore provided with a pair of lower sealing members 24 that are formed integrally with the sealing flange 20, and extend inwardly from the lower edge of the mask body 10 (as viewed in Figures 3 and 4). The lower sealing members 24 are also resilient in form, so that when the contact surface of the respiratory mask is urged against the patient's face, the lower sealing members 24 are deformed, and therefore urged against, the surface of the patient's chin. The lower sealing members 24 are connected directly together by a narrow connecting portion but are separated by a longitudinal slit that extends from the connecting portion to the upper edge of the lower sealing members 24. This slit allows the lower sealing members 24 to be deformed in different directions and therefore urged against both sides of the patient's chin. In this way, the lower sealing members 24 provide an additional seal between the respiratory mask and the face of the patient. In addition, the slit allows the lower sealing members 24 to deform on removal from the mould, during manufacture, without being damaged.

The lower sealing members 24 may therefore allow a particular size of respiratory mask to fit faces having a greater range of sizes than has hitherto been possible.

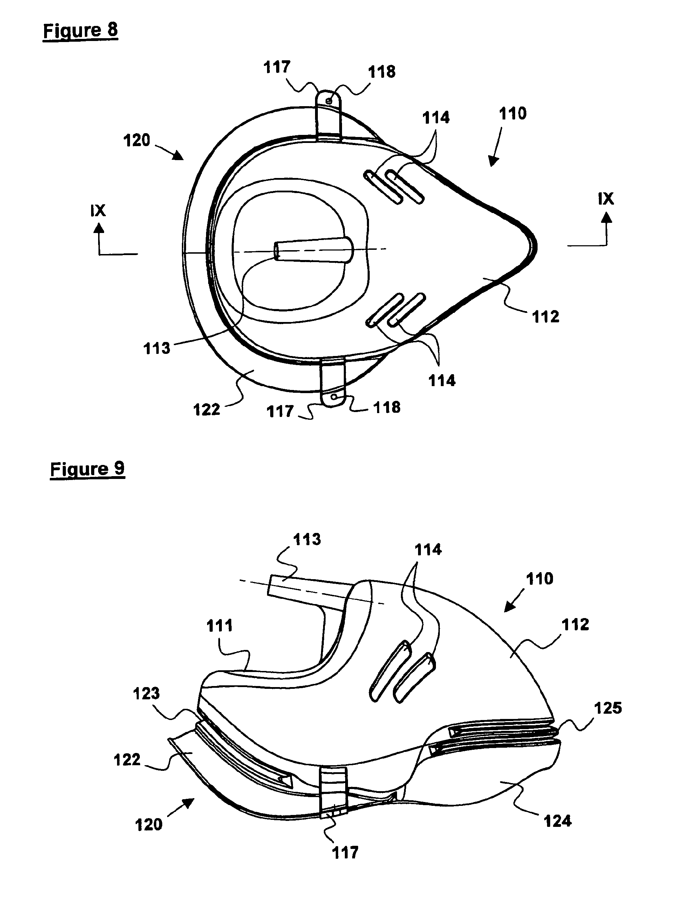

Figures 8 to 11 each show a second respiratory mask, which represents an embodiment of the present invention. The second mask is of similar overall construction to the first mask, and comprises a mask body 110, formed from a suitably strong and relatively rigid plastics material, such as polypropylene, and a sealing component 120 formed from a Styrene-Ethylene-Butylene-Styrene (SEBS)-based thermoplastic elastomer.

The second embodiment is manufactured using a so-called two-shot injection moulding process. In particular, the mask body 110 is firstly injection moulded as a single component, and the sealing component 120 is then injection moulded onto the surface of the mask body 110. The mask body 110 and the sealing component 120 are bonded together by this process.

The mask body 110 defines a cavity from which an inhalation gas is delivered to a patient, and comprises a mouth portion 111 and a nose portion 112 that each have a similar form to the mouth portion 11 and nose portion 12 of the first embodiment.

The sealing component 120 is a unitary component that is bonded to, and extends from, the peripheral edge of the mask body 110. The sealing component 120 comprises a chin portion 122 and a nose portion 124 that together define a continuous contact surface that is urged against the face of the patient during use. The elastomeric nature of the sealing component 120 enables an effective seal to be formed between the contact surface of the respiratory mask and the face of the patient.

The chin portion 122 of the sealing component 120 comprises a connecting member 123 that extends from the peripheral edge of the mask body 120 to a sealing member that defines the portion of the contact surface that extends about the mouth portion 111 of the mask body 110.

The sealing member of the chin portion 122 extends from the connecting member 123 in both an inward and an outward direction relative to the peripheral edge of the mask body 110. The inward extension of the sealing member reduces gradually in size from a maximum at the end furthest from the nose portion 124 to a minimum at the interface between the chin and nose portions 122,124 of the sealing component 120 on either side of the mask. The sealing member therefore partially occludes the opening defined by the edge of the mask body 110.

The portion of the contact surface defined by the sealing member of the chin portion 122 is substantially flat in form, but will deform into conformity with the contours of the patient's face when the sealing member 120 is urged against the patient's face during use. In this way, an effective seal is provided between the chin portion 122 of the sealing member 120 and the face of the patient.

The sealing member of the chin portion 122 provides an improved seal between the respiratory mask and the face of the patient relative to sealing members of conventional respiratory masks. In addition, the greater contact surface area of the chin portion 122 relative to conventional masks may allow a particular size of respiratory mask to be used with faces having a greater range of shapes and sizes of anatomical features than has hitherto been possible.

The connecting member 123 is convoluted in form so that it is extendible in directions away from, or towards, the peripheral edge of the mask body 110. This enables the sealing member of the chin portion 122 to be moved relative to the mask body 110 during use. In this way, the patient can talk whilst wearing the mask, and the sealing member will move with the patient's mandible, thereby improving patient comfort.

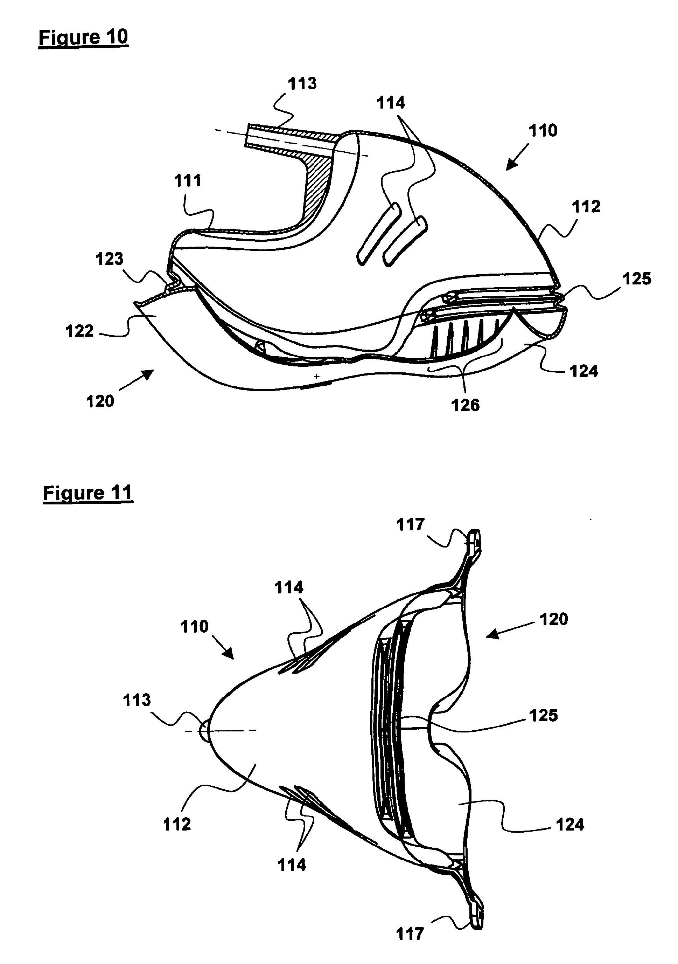

The nose portion 124 of the sealing component 120 comprises a connecting member 125 that extends from the peripheral edge of the mask body 110 to a sealing member that defines the portion of the contact surface that extends about the nose portion 112 of the mask body 110. The sealing member of the nose portion 124 is arcuate in form, and extends from the connecting member 125 in an inward direction relative to the peripheral edge of the mask body 110. The sealing member therefore partially occludes the opening defined by the edge of the mask body 110. As shown most clearly in Figure 11, the sealing member is formed with a depressed portion that is formed so as to accommodate the bridge of the patient's nose.

The portion of the contact surface defined by the sealing member of the nose portion 124 is convex in form, but will deform into conformity with the contours of the patient's face when the sealing member 120 is urged against the patient's face during use. In this way, an effective seal is provided between the nose portion 124 of the sealing member 120 and the face of the patient.

The sealing component 120 is formed with the sealing members of the chin and nose components 122,124 joining such that the contact surface defined by the chin and nose portions 122,124 of the sealing member 120 is continuous. The connecting member 125 has a concertina-type configuration, as shown most clearly in Figure 10, so that it is more readily deformable than the sealing member. This enables the nose portion 124 of the sealing component 120 to seal against different sizes and shapes of nose without excessive deformation, and hence lessened sealing properties, of the sealing member.

In addition, a series of reinforcing ribs 126 is formed on the inner surface of the sealing member of the nose portion 124, either side of the depressed portion for accommodating the bridge of the nose. The reinforcing ribs 126 are orientated generally perpendicularly relative to the peripheral edge of the mask body 110, and give the parts of the nose portion 124 in which the reinforcing ribs are formed more strength. This causes the other parts of the nose portion 124, and in particular the parts of the nose portion 124 that seal against the bridge of a patient's nose during use, to deform to a greater extent than the re-enforced parts of the nose portion 124. In this way, an improved seal can be formed between the nose portion 124 of the sealing component 120 and the face of the patient.

The form of the nose portion 124 of the sealing component 120 allows the second embodiment of the respiratory mask according to the invention to be used without a nose clip, allowing the mask to be fitted quicker and more easily to the patient.

The mask body 110 further comprises an inlet port 113 for connection to a supply of an inhalation gas, and a pair of exhalation openings 114. The inlet port 113 comprises an opening in a lower wall of the nose portion 112, and a tubular connector that extends from this opening into the space in front of the mouth portion 111. The tubular connector of the inlet port 113 is of reduced diameter relative to that of the first embodiment. In particular, the first mask is an aerosol respiratory mask with a 22mm tubular connector, and the second mask is a medium concentration oxygen mask with a 6mm tubular connector. In use, a supply of an inhalation gas is connected to the tubular connector of the inlet port 113 so as to supply the inhalation gas to the cavity of the respiratory mask and hence the airways of the patient. The exhalation openings 114 are elongated openings in the wall of the mask body 110 that allow exhaled gases to exit the cavity of the respiratory mask, and also allow the intake of air for inhalation, as required, when the inhalation volume exceeds the volume of the mask cavity and the inlet flow.

The chin portion 122 of the sealing component 120 is formed with a pair of outwardly extending wings 117, on either side of the respiratory mask, that each have an aperture 118 to which an elasticated strap (not shown in the Figures) is attached, in use. The elasticated strap extends between the pair of wings 117, and fits around the patient's head when the respiratory mask is fitted to the patient. In use, the elasticated strap is adjusted so that the respiratory mask is urged against the face of the patient with an appropriate force to ensure that an effective seal is formed between the contact surface of the respiratory mask and the patient's face, without causing excessive discomfort for the patient.

## Claims

1. A respiratory mask for delivering an inhalation gas to the airways of a patient, the respiratory mask comprising a mask body (110) defining a cavity and being adapted to fit about the mouth and nose of the patient such that the inhalation gas can be inhaled by the patient from the cavity, an inlet port (113) that is engageable with a supply of inhalation gas and in communication with the cavity, and one or more sealing components (120) extending from the peripheral edge of the mask body (110) and defining a contact surface that contacts the nose of the patient during use, one or more of the sealing components comprising a sealing member (122,124) that defines the contact surface and a connecting member (123,125) that connects the sealing member to the peripheral edge of the mask body (110),
**characterised in that** the one or more sealing members (124) include reinforcing formations (126) that reduce the deformability of those parts of the sealing member in which they are formed, wherein the connecting member is resilient in form and is more deformable, in use, than the sealing member.

2. A respiratory mask as claimed in Claim 1, wherein the connecting member (123,125) is formed of the same material as the sealing member, and the connecting member has a configuration that provides the greater deformability.

3. A respiratory mask as claimed in Claim 2, wherein the connecting member (125) is convoluted in form.

4. A respiratory mask as claimed in Claim 3, wherein the connecting member (125) has a corrugated or concertina-type configuration.

5. A respiratory mask as claimed in Claim 1, wherein reinforcing formations (126) are provided on the parts of one or more sealing members (122,124) that contact either side of a patient's nose so that the parts of one or more sealing members that contact the bridge of a patient's nose deform to a greater degree.

6. A respiratory mask according to Claim 1, wherein the reinforcing formations (126) comprise reinforcing ribs.

7. A respiratory mask according to Claim 1, wherein the sealing component (120) is a unitary component that is bonded to, and extends from, the peripheral edge of the mask body (110).

8. A respiratory mask according to Claim 7, wherein the sealing component (120) comprises a chin portion (122) and a nose portion (124) that together define the continuous contact surface that is urged against the face of the patient during use.

## Patentansprüche

1. Atemmaske zum Zuführen eines Inhalationsgases zu den Atemwegen eines Patienten, wobei die Atemmaske Folgendes aufweist: einen Maskenkörper (110), der einen Hohlraum definiert und so ausgeführt ist, dass er so um den Mund und die Nase des Patienten passt, dass das Inhalationsgas von dem Patienten aus dem Hohlraum eingeatmet werden kann, einen Einlasskanal (113), der mit einer Inhalationsgasversorgung in Eingriff gebracht werden kann und mit dem Hohlraum in Kommunikation ist, und eine oder mehrere Dichtungskomponenten (120), die sich von dem Umfangsrand des Maskenkörpers (110) erstrecken und eine Kontaktfläche definieren, die während des Gebrauchs mit der Nase des Patienten in Kontakt ist, wobei eine oder mehrere der Dichtungskomponenten ein Dichtungselement (122, 124) aufweisen, das die Kontaktfläche und ein Verbindungselement (123, 125) definiert, welches das Dichtungselement mit dem Umfangsrand des Maskenkörpers (110) verbindet,
**dadurch gekennzeichnet, dass** das eine oder die mehreren Dichtungselemente (124) Verstärkungsgebilde (126) beinhalten, die die Verformbarkeit jener Teile des Dichtungselements verringern, in denen sie ausgebildet sind, wobei das Verbindungselement nachgiebig geformt ist und im Gebrauch verformbarer ist als das Dichtungselement.

2. Atemmaske nach Anspruch 1, wobei das Verbindungselement (123, 125) aus dem gleichen Material wie das Dichtungselement hergestellt ist und das Verbindungselement eine Ausgestaltung hat, die die größere Verformbarkeit bereitstellt.

3. Atemmaske nach Anspruch 2, wobei das Verbindungselement (125) eine gefaltete Form hat.

4. Atemmaske nach Anspruch 3, wobei das Verbindungselement (125) eine gewellte oder faltenbalgartige Ausgestaltung hat.

5. Atemmaske nach Anspruch 1, wobei die Verstärkungsgebilde (126) an den Teilen von einem oder mehreren Dichtungselementen (122, 124) bereitgestellt sind, die mit den beiden Seiten der Nase eines Patienten in Kontakt sind, so dass die Teile von einem oder mehreren Dichtungselementen, die mit dem Nasensteg eines Patienten in Kontakt sind, sich in einem größeren Maße verformen.

6. Atemmaske nach Anspruch 1, wobei die Verstärkungsgebilde (126) Verstärkungsrippen aufweisen.

7. Atemmaske nach Anspruch 1, wobei die Dichtungskomponente (120) eine einstückige Komponente ist, die mit dem Umfangsrand des Maskenkörpers (110) festverbunden ist und sich von ihm erstreckt.

8. Atemmaske nach Anspruch 7, wobei die Dichtungskomponente (120) einen Kinnabschnitt (122) und einen Nasenabschnitt (124) aufweist, die zusammen die durchgehende Kontaktfläche definieren, die während des Gebrauchs gegen das Gesicht des Patienten gedrängt wird.

## Revendications

1. Masque respiratoire pour distribuer un gaz d'inhalation aux voies respiratoires d'un patient, le masque respiratoire comprenant un corps de masque (110) définissant une cavité et étant adapté pour s'ajuster autour de la bouche et du nez du patient de telle sorte que le gaz d'inhalation puisse être inhalé par le patient à partir de la cavité, un orifice d'entrée (113) qui peut être mis en prise avec un approvisionnement en gaz d'inhalation et en communication avec la cavité, et un ou plusieurs composants d'étanchéité (120) s'étendant à partir du bord périphérique du corps de masque (110) et définissant une surface de contact qui entre en contact avec le nez du patient pendant l'utilisation, un ou plusieurs des composants d'étanchéité comprenant un élément d'étanchéité (122,124) qui définit la surface de contact et un élément de connexion (123,125) qui connecte l'élément d'étanchéité au bord périphérique du corps de masque (110),
**caractérisé en ce que** l'un ou plusieurs éléments d'étanchéité (124) incluent des formations de renforcement (126) qui réduisent la déformabilité de ces parties de l'élément d'étanchéité dans lesquelles elles sont formées, où l'élément de connexion est de forme élastique et est davantage déformable, lors de l'utilisation, que l'élément d'étanchéité.

2. Masque respiratoire tel que revendiqué dans la Revendication 1, où l'élément de connexion (123,125) est formé du même matériau que l'élément d'étanchéité, et l'élément de connexion a une configuration qui fournit la plus grande déformabilité.

3. Masque respiratoire tel que revendiqué dans la Revendication 2, où l'élément de connexion (125) est de forme vrillée.

4. Masque respiratoire tel que revendiqué dans la Revendication 3, où l'élément de connexion (125) a une configuration ondulée ou de type en accordéon.

5. Masque respiratoire tel que revendiqué dans la Revendication 1, où des formations de renforcement (126) sont prévues sur les parties d'un ou de plusieurs éléments d'étanchéité (122,124) qui entrent en contact avec l'un ou l'autre côté du nez d'un patient de sorte que les parties d'un ou de plusieurs éléments d'étanchéité qui entrent en contact avec l'arête du nez d'un patient se déforment dans une plus grande mesure.

6. Masque respiratoire selon la Revendication 1, où les formations de renforcement (126) comprennent des bourrelets de renforcement.

7. Masque respiratoire selon la Revendication 1, où le composant d'étanchéité (120) est un composant unitaire qui est lié au bord périphérique du corps de masque (110) et s'étend à partir de celui-ci.

8. Masque respiratoire selon la Revendication 7, où le composant d'étanchéité (120) comprend une partie de menton (122) et une partie de nez (124) qui définissent ensemble la surface de contact continue qui est pressée contre le visage du patient pendant l'utilisation.
